# EUROPEAN PATENT SPECIFICATION

(11) **EP 3 797 755 B1**
(45) Date of publication and mention of the grant of the patent: **13.08.2025**
(21) Application number: 19199272.6
(22) Date of filing: 24.09.2019
(51) Int. Cl.: A61K 8/02, A61K 8/34, A61Q 11/00

(54) **POWDER AND PROCESS FOR CLEANING TEETH BY USE OF SUCH POWDER**
PULVER UND VERFAHREN ZUR ZAHNREINIGUNG UNTER VERWENDUNG DIESES PULVERS
POUDRE ET PROCÉDÉ DE NETTOYAGE DES DENTS AU MOYEN D'UNE TELLE POUDRE

(43) Date of publication of application: 31.03.2021
(73) Proprietor: Ferton Holding S.A., 2800 Delémont (CH)
(72) Inventor: Donnet, Marcel, 01630 Saint Jean de Gonville (FR); Gatti, Simone, 1022 Chavannes-près-Renens (CH)
(74) Representative: Müller Schupfner & Partner Patent- und Rechtsanwaltspartnerschaft mbB (Muc)

(56) References cited:
- DE-A1- 102015 201 871
- DE-T2- 3 786 754
- GB-A- 2 252 042
- US-A1- 2003 031 632
- US-A1- 2013 337 413

## Description

The invention relates to a powder for use in a powder jet device for cleaning a tooth surface by powder spraying. The invention further relates to a powder for use in cleaning a tooth surface by powder spraying with a powder jet device.

Powder jet cleaning or air polishing is a dental prophylaxis method that is particularly effective since it allows to reach and to clean all the teeth surfaces and the interspaces between teeth, as well as implants, brackets and appliances.

The powder is sprayed together with a gaseous carrier medium, usually air, onto the tooth surface, thereby achieving an efficient cleaning of a tooth surface. Additionally, or as an alternative to a gaseous carrier medium, a liquid carrier medium, for example water, may in principle also be used. This occurs without damaging enamel, dentine and soft tissues as long as the powder is sufficiently soft and particles are sufficiently small.

A dental cleaning powder is described for example in DE 200 09 665 U1. The powder contains a basic powder of sodium bicarbonate, alternatively calcium carbonate or glycine and additional active ingredients such as an anti-microbial compound or an ingredient which contributes to the remineralization of the teeth.

Another powder for use in a powder jet device is described in EP 2 228 175 A1, wherein the powder is based on alditols, in particular mannitol and/or erythritol.

It is known from EP 3 253 359 A1 that the particles should have an average grain size of 10 µm to 100 µm for a gentle and nevertheless efficient tooth cleaning, in particular an average grain size of 10 µm to 65 µm. A smaller grain size such as approximately 10 µm to 30 µm is preferred for cleaning subgingival tooth surfaces. For tooth cleaning of the supragingival tooth surfaces, a larger grain size, in particular approximately 40 µm to 65 µm, is usually preferred. Grain size or particle size as used herein usually refer to an average grain size or an average particle size of a respective powder mixture.

Powders with small average particle sizes allow gentle and efficient tooth cleaning, but such powders lead to several problems. First of all, a high dust generation occurs during handling, for example when the device is filled. Further, a high precision of device filling is often not possible because powders with small average particle sizes show large differences in the apparent densities when they are free flowing or when there are compacted. Consequently, filling up the cleaning device to the maximum level is complicated because the powder level varies over time due to the slow powder compaction. Another problem which arises from the apparent density variation is that filling up of the bottles during production is cumbersome and usually requires to fill up bottles only with half of the volume due to the maximum level variation. The above results in handling difficulties for the user as well as the manufacturer. On contrary, powders with bigger grain sizes which do not have these limitations cannot be used for subgingival application because they are too abrasive and the impact of the bigger particles is felt on the soft tissues (gum) and hurts.

DE 37 86 754 T2 discloses a granulated composition and its use in toothpastes comprising a powder and an inorganic binder, wherein the powder and the inorganic binder are both water-insoluble.

GB 2 252 042 A discloses a composition for use in the oral cavity comprising granules having a particle size in the range of 0.1 to 20 µm, wherein the granules comprise smaller particles and a water-insoluble organic binder.

US 2003/031632 A1 discloses a powder with an average granule size of 50 - 500 µm, wherein the granules comprise primary particles and a binder, wherein the primary particles are made of calcium carbonate.

In view of the above, it is the object of the present invention to provide a powder for use in a powder jet device for cleaning teeth that overcomes the afore-mentioned disadvantages of conventional powders, in particular dust formation and powder compaction difficulties, and still allows an efficient and gentle cleaning of teeth. A further object of the invention is to provide a corresponding method or process for cleaning teeth.

This object is achieved according to the invention by a powder (dental cleaning powder) for use in a powder jet device for cleaning a tooth surface by powder spraying, wherein the powder comprises granules having an average particle size (average granule size) of 20 µm to 220 µm, and wherein the granules comprise primary particles and a binder, the primary particles having an average particle size that is smaller than the average particle size of the granules and wherein the primary particles are made of a material selected from the group consisting of salts that are soluble in water, sugars, amino acids, alditols, except calcium carbonate, and mixtures thereof.

The object is also achieved by a powder for use in cleaning a tooth surface by powder spraying with a powder jet device according to claim 11.

Preferred embodiments of the invention are subject of the dependent claims as well as the following description.

The invention combines advantages of two opposite effects, namely the advantageous effects of large and small particles. This is achieved by binding small particles (herein called primary particles) to larger particles (herein called granules). The granules are present during handling before the cleaning process starts, i.e. during filling the powder bottles in the powder production and filling up the powder jet device for teeth cleaning, whereas the primary particles or at least fragments of the granules are formed in or in the vicinity of the nozzle of the powder jet device or when the granules fly towards or hit the tooth surface by fragmentation of the granules. As a result, the cleaning effect, particularly the abrasivity, corresponds to the primary particles (small particles) and the handling corresponds to the granules (large particles).

Very surprisingly, the abrasivity of the powders according to the invention is even lower than the abrasivity of the primary particles alone. Without being bound by this explanation, it is assumed that this positive effect is achieved by the binder that is sufficiently soft to allow breaking of the granules into particles and that is still present at parts of the particle surfaces that are responsible for the abrasivity and the cleaning effect.

The granules preferably have an average particle size (average granule size d_{50,g}) of 20 µm to 180 µm, more preferably of 25 µm to 150 µm, even more preferably of 25 µm to 120 µm and most preferably of 30 µm to 100 µm. Granules in this size range allow an efficient dust-free handling and avoid problems resulting from a slow compaction of powders consisting of fine particles.

For a gentle and nevertheless efficient tooth cleaning, the primary particles, forming the granules together with the binder, preferably have an average particle size (d_{50,p}) of 5 µm to 75 µm, more preferably 10 µm to 70 µm. The particle sizes may be adapted to the field of application of the dental cleaning powder that is provided. For example, a smaller average particle size of the primary particles is preferred for cleaning subgingival tooth surfaces, in particular approximately 5 µm to 40 µm, preferably 10 µm to 30 µm. For tooth cleaning of the supragingival tooth surfaces, a larger average particle size of the primary particles is preferred, in particular approximately 20 µm to 75 µm, more preferably 30 µm to 50 µm.

In the context of this invention, the d₅₀ value is the granule size or particle size at which 50% of the particles are smaller than the d₅₀ value in terms of volume and 50% of the particles are larger than the d₅₀ value in terms of volume. The d₅₀ value is determined by laser diffraction using a dry dispersion unit. Particular attention should be paid to use only a small dispersion air pressure, in particular 0.5 bar, when measuring the average granule size in order to not break the fragile granules before the measurement. The instrument that is used to measure average particle sizes is the Malvern Mastersizer 2000 equipped with a Sirocco dry dispersion unit and operated with 0.5 bars dispersion pressure. The measurement method is further described in the experimental section.

The binder binds the primary particles within the granules. In a preferred embodiment of the invention, the binder is a dietary fibre, a polysaccharide, a synthetic polymer, a salt, a sugar, an alditol, an amino acid, the same material as the material of the primary particles, or mixtures thereof. They allow a soft binding of the particles so that an efficient breakage or fragmentation of the granules into smaller fragments is possible in usual powder jet devices for tooth cleaning.

The fragmentation is usually a partial fragmentation, wherein preferably 50 to 99.9 % of the granules break into fragments in the nozzle of powder jet devices and/or when the powder hits the tooth surface (50 to 99.9 % fragmentation). A fragmentation of 90 to 99.9 % is more preferred. For measuring the corresponding size reduction after fragmentation there are two possibilities. To measure the size reduction between two different dispersing pressures in a laser diffraction unit such as Malvern Mastersizer with Scirocco unit or to measure the size reduction of the powder after passing through the nozzle of a powder jet device (air-polishing device) such as EMS Airflow^{®} prophylaxis master. The details are described in the experimental section.

In a preferred embodiment of the invention, the material of the binder is one or more selected from the group consisting of cellulose, hemicellulose, cellulose derivatives, maltodextrins, corn dextrins, Xanthan gum, guar gum and gum arabic. A trade name of a preferred corn dextrin is Nutriose^{®}. These binders provide a soft binding that gives an efficient fragmentation in the powder jet device, in particular in the nozzle and/or when the granules and their potential fragments hit the tooth surface.

The powder according to the invention can be prepared by a process comprising the steps that a binder is mixed with water, the resulting mixture is sprayed on the primary particles and the particles are dried to form granules. Preferably, the powder is prepared by using a fluidized bed granulator. The binder is mixed with water, sprayed onto a powder fluidized bed and dried. The drying can be conducted at room temperature or elevated temperature. The liquid addition rate and the temperature are chosen so that the binding solution falls on the primary particles and dries out, gluing the primary particles together to form the granules. Granulation parameters are optimized according to the specific binder solution.

In view of the fact that there is a partial fragmentation of the granules, the average particle size of the powder after fragmentation in the nozzle and/or on the tooth surface is lower than the average particle size of the granules. The average particle size of the powder after breaking or fragmentation (d_{50,p-b}) depends on the fragmentation degree and the particle size of additional components in the powder.

The powder after fragmentation or breakage preferably has an average particle size d_{50,p-b} of 5 µm to 75 µm, in particular 10 µm to 70 µm. Preferred size ranges depend on the field of application of the dental cleaning powder that is provided. Smaller average particle sizes are preferred for cleaning subgingival tooth surfaces, and larger average particle sizes are preferred for cleaning supragingival tooth surfaces. The powder for cleaning subgingival tooth surfaces preferably has an average particle size of 5 µm to 40 µm after fragmentation, more preferably 10 µm to 30 µm. The powder for cleaning supragingival tooth surfaces after fragmentation preferably has an average particle size of 20 µm to 75 µm, more preferably 30 µm to 50 µm.

The granules comprise preferably 80 - 99.5 % by weight of the particles and 0.5 - 20 % by weight of the binder, in particular 90 - 99.5 % by weight of the particles and 0.5 - 10 % by weight of the binder.

The particles are made of substances suitable for dental cleaning purposes. Suitable materials are chosen depending on the field of application of the dental cleaning powder, for example powder for cleaning supragingival tooth surfaces or cleaning subgingival tooth surfaces. The particles are made of water-soluble salts, alditols, amino acids, sugars, except calcium carbonate, and mixtures thereof. Soluble salts can be organic or inorganic salts, in particular sodium hydrogen carbonate, alditols can be mannitol or erythritol, amino acids can be glycine and sugars can be trehalose or tagatose. The particles are not made of calcium carbonate. The material of the particles of the powder according to invention is more preferably one or more selected from the group consisting of mannitol, erythritol, glycine, trehalose, tagatose, and sodium hydrogen carbonate. These substances in the form of powders provide moderate abrasivity for an effective cleaning without destroying the tooth surface.

The primary particles preferably contain at least 80 weight-%, more preferably at least 90 weight-% of the above-mentioned substances, for example sodium hydrogen carbonate, alditols, mannitol, erythritol, glycine, trehalose, tagatose or mixtures thereof.

In a further preferred embodiment of the invention, the binder and the primary particles are made of the same material. When the materials of the binder and the primary particles are the same, preferred materials are the preferred materials of the primary particles, in particular soluble salts, alditols, amino acids and sugars as well as mixtures thereof. "Soluble" means soluble in water. According to one embodiment of the invention the material is not calcium carbonate. That means the materials of the primary particles and/or the binder are preferably soluble salts except calcium carbonate, alditols, amino acids, sugars and mixtures thereof. More preferred are one or more compounds selected from the group consisting of mannitol, erythritol, glycine, trehalose, tagatose, and sodium hydrogen carbonate. In case the binder is of the same material as the primary particles, the binder can be distinguished from the primary particles as the binder is not in particulate form, but functions as binder between the primary particles.

The powder according to the invention may optionally contain additives such as silica gel, bleaching agents, analgetics, bacteriocides and/or flavour additives. These additives may be admixed in the binder solution before preparing the granules (granulation step) to become part of the granules, at least partially, or the additives may be added after forming the granules to become part of the powder in form of additional particles. It is preferred that additives are added in the binder solution to be part of the granules.

It is also described a process for cleaning teeth, wherein the powder is sprayed through a nozzle onto a tooth surface together with a gaseous carrier medium, in particular air and optionally a fluid, such as water, for cleaning the tooth surface. In a preferred process, the granules break in the nozzle or in the vicinity of the nozzle of the powder jet device or when hitting the tooth surface during the cleaning process. The inventive powder can be used with common powder jet devices.

The invention also relates to a powder for use in cleaning teeth in a powder jet cleaning device for cleaning a tooth surface by powder spraying.

### Examples

The following examples provide preferred powders according to the invention.

### Example 1

### Erythritol granulated with Nutriose^{®}

The experiment was carried out in a pilot batch fluidized bed of conical shape. Initial particle mass 1 kg, inlet air temperature 80 °C, liquid feed rate 10 ml min⁻¹ and relative air spraying pressure 1.5 bar were chosen as process parameters. A solution of 5% Nutriose^{®} was sprayed for 20 min. During the experiment, all process parameters (air inlet temperature, air flow rate, liquid feed rate and spraying pressure) were kept constant. At the end of the experiment the granulated powder was removed and the solid content checked to be more than 98%.

### Example 2

### Erythritol granulated with Cellulose

The experiment was carried out in a pilot batch fluidized bed of conical shape. Initial particle mass 1 kg, inlet air temperature 80 °C, liquid feed rate 8.6 ml min⁻¹ and relative air spraying pressure 1.5 bar were chosen as process parameters. A solution of 7% Methyl cellulose^{®} was sprayed for 15 min. During the experiment, all process parameters (air inlet temperature, air flow rate, liquid feed rate and spraying pressure) were kept constant. At the end of the experiment the granulated powder was removed and the solid content checked to be more than 98%.

### Example 3

### Erythritol granulated with Maltodextrin

The experiment was carried out in a pilot batch fluidized bed of conical shape. Initial particle mass 1 kg, inlet air temperature 80 °C, liquid feed rate 10 ml min⁻¹ and relative air spraying pressure 1.5 bar were chosen as process parameters. A solution of 20% Maltodextrin was sprayed for 20 min. During the experiment, all process parameters (air inlet temperature, air flow rate, liquid feed rate and spraying pressure) were kept constant. At the end of the experiment the granulated powder was removed and the solid content checked to be more than 98%.

### Example 4

### Erythritol granulated with Gum Arabic

The experiment was carried out in a pilot batch fluidized bed of conical shape. Initial particle mass 1 kg, inlet air temperature 80 °C, liquid feed rate 7 ml min⁻¹ and relative air spraying pressure 1.5 bar were chosen as process parameters. A solution of 1% Gum arabic was sprayed for 30 min. During the experiment, all process parameters (air inlet temperature, air flow rate, liquid feed rate and spraying pressure) were kept constant. At the end of the experiment the granulated powder was removed and the solid content checked to be more than 98%.

### Example 5

### Abrasiveness on Aluminium surface

Abrasiveness is tested by projecting powder with a nozzle directly on a surface at 45° and 2mm of projected distance using an EMS Airflow^{®} prophylaxis master device. This surface is made of pure aluminium (99.5%). The application time is 30 seconds. The plate is put on an elevator to reach the distance of 2 mm. The nozzle is fixed in a resin mould in order to fix well the nozzle position. The mass is known by weighing the powder chamber before and after the test. Every measurement is repeated at least three times. The depth of the holes was measured by a laser profilometer.

### Measurement of average particle sizes and size reduction

To measure the size reduction there are two possibilities:
1) To measure the average particle size of the powder with a laser diffraction unit, of a Malvern Mastersizer with Scirocco dispersing unit, at 0.5 bar and 1.5 bar. Here the size reduction can be defined as: (1-(d₅₀ at 1.5bar/d₅₀ at 0.5bar))x100 %
2) To measure the average particle size of the powder in a Malvern Mastersizer with Scirocco dispersing unit at 0.5 bar (average granule size) and to measure the average particle size after the nozzle of a powder jet device by spraying the powder directly in the laser diffraction unit using an air polishing device such as EMS Airflow^{®} prophylaxis master at maximum pressure (average particle size after nozzle). Here the size reduction can be defined as (1-(d₅₀ after nozzle/d₅₀ at 0.5bar))x100 %.

The size reduction of the powder according to the invention, defined by (1-(d₅₀ after nozzle/d₅₀ at 0.5bar))x100 %, is preferably at least 20 %, more preferably at least 30 %, even more preferably at least 35 % and most preferably 35 to 75%. The size reduction of the powder according to the invention, defined by (1-(d₅₀ at 1.5bar/d₅₀ at 0.5bar))x100 %, is preferably at least 20 %, more preferably at least 30 %, most preferably 30 to 70%.

Table 1 shows the trend of average particle size of three different powders, in accordance with Examples 1 to 3, in function of the dispersing pressure of a laser diffraction measurement unit. The average particle size at 0.5 bar is the average particle size of the granules.

**Table 1. Average particle size (µm) in function of the dispersing pressure**

| | Erythritol granulated with Nutriose^{®} | Erythritol granulated with cellulose | Erythritol granulated with maltodextrin |
|---|---|---|---|
| d₅₀ at P=0.5 Bar | 91.8 µm | 143.0 µm | 150.4 µm |
| d₅₀ at P=1.0 Bar | 71.3 µm | 92.3 µm | 110.5 µm |
| d₅₀ at P=1.5 Bar | 61.5 µm | 85.9 µm | 101.9 µm |
| d₅₀ at P=2.5 Bar | 38.8 µm | 65.0 µm | 78.4 µm |

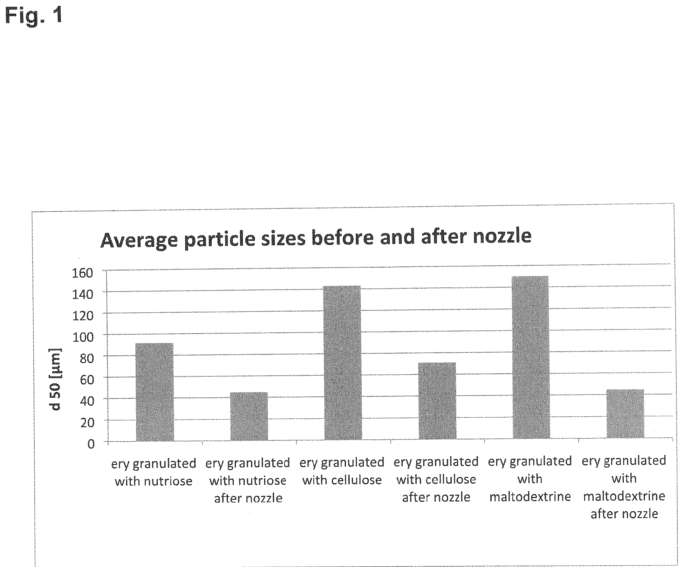

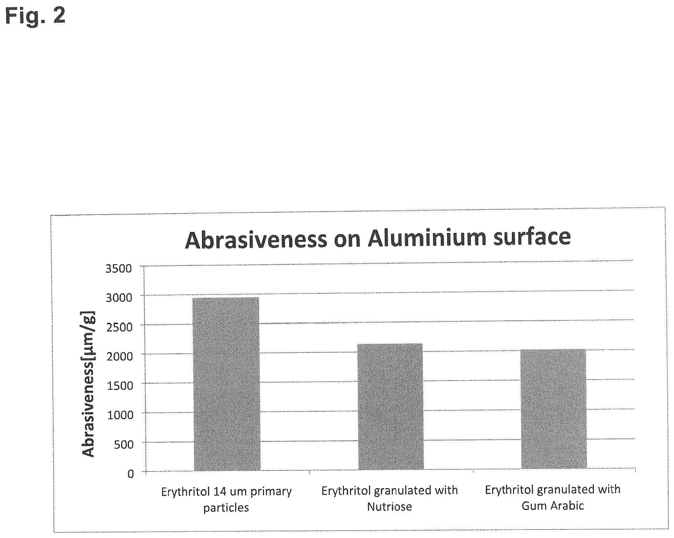

The invention is described further below with reference to Figures 1 and 2 (FIG. 1 and 2).

FIG. 1 shows the average particle sizes before the nozzle (average granule sizes d_{50,g}) and the average particle sizes after the nozzle (average particle sizes after breaking d_{50,p-b}) of three different powders (erythritol granulated with Nutriose^{®}, cellulose, and maltodextrin) in accordance with Examples 1 to 3 when using them in an Airflow^{®} prophylaxis master. The average particle sizes before the nozzle are about 90 µm to 150 µm and average particle sizes after breaking are about 45 µm to 70 µm.

FIG. 2 shows the abrasiveness on an aluminium surface in accordance with Example 5. The abrasiveness on an aluminium surface of the two different powders in accordance with the invention (erythritol granulated with Nutriose^{®} and Gum Arabic) are compared with erythritol particles without binder. The abrasiveness of the powders according to the invention is significantly lower than the abrasiveness of erythritol primary particles, i.e. erythritol particles without binder.

It has been demonstrated that the powders according to the invention have average granule sizes sufficiently high to avoid the handling difficulties of small particles and simultaneously provide particle sizes at the tooth surface that allow a gentle and efficient cleaning of teeth, including cleaning subgingival tooth surfaces.

## Claims

1. Powder for use in a powder jet device for cleaning a tooth surface by powder spraying, wherein the powder comprises granules having an average particle size of 20 µm to 220 µm, **characterized in that** the granules comprise primary particles and a binder, wherein the primary particles have an average particle size smaller than the average particle size of the granules, **characterized in that** the primary particles are made of a material selected from the group consisting of salts that are soluble in water, sugars, amino acids, alditols, except calcium carbonate, and mixtures thereof.

2. The powder according to claim 1, **characterized in that** the average particle size of the granules is 25 µm to 150 µm.

3. The powder according to claim 1 or 2, **characterized in that** the binder is a dietary fibre, a polysaccharide, a synthetic polymer, a salt, a sugar, an amino acid, the same material as the material of the primary particles, or mixtures thereof.

4. The powder according to one of the claims 1 to 3, **characterized in that** the binder is made of a material selected from the group consisting of cellulose, hemicellulose, methyl cellulose, cellulose derivatives, maltodextrins, corn dextrins, gum arabic, gum xanthane, guar gum and mixtures thereof.

5. The powder according to one of the claims 1 to 4, **characterized in that** the granules comprise 80 - 99.5 % by weight of the particles and 0.5 - 20 % by weight of the binder.

6. The powder according to one of the claims 1 to 5, **characterized in that** the average particle size of the primary particles is 5 µm to 40 µm.

7. The powder according to one of the claims 1 to 6, **characterized in that** the granules break during powder spraying into fragments.

8. The powder according to claim 7, **characterized in that** the powder after the breaking has an average particle size of 10 µm to 75 µm.

9. The powder according to one of the claims 1 to 8, **characterized in that** the size reduction in a powder jet device, defined by (1-(d₅₀ after nozzle/d₅₀ at 0.5bar))x100 %, is at least 20 %.

10. The powder according to one of the claims 1 to 9, **characterized in that** the primary particles are made of a material selected from the group consisting of sodium hydrogen carbonate, mannitol, erythritol, glycine, trehalose, tagatose, and mixtures thereof.

11. Powder for use in cleaning a tooth surface by powder spraying with a powder jet device, wherein the powder comprises granules having an average particle size of 20 µm to 220 µm, **characterized in that** the granules comprise primary particles and a binder, wherein the primary particles have an average particle size smaller than the average particle size of the granules.

12. Process for preparing a powder according to one of the claims 1 - 10, comprising the steps that a binder is mixed with water, the resulting mixture is sprayed onto the primary particles and the particles are dried to form the granules.

## Patentansprüche

1. Pulver zur Verwendung in einem Pulverstrahlgerät zur Reinigung einer Zahnoberfläche durch Pulversprühen, wobei das Pulver Granulate mit einer durchschnittlichen Partikelgröße von 20 µm bis 220 µm aufweist, **dadurch gekennzeichnet, dass** die Granulate Primärpartikel und ein Bindemittel aufweisen, wobei die Primärpartikel eine durchschnittliche Partikelgröße aufweisen, die kleiner ist als die durchschnittliche Partikelgröße der Granulate, **dadurch gekennzeichnet, dass** die Primärpartikel aus einem Material hergestellt sind, das aus der Gruppe ausgewählt ist, die aus wasserlöslichen Salzen, Zuckern, Aminosäuren, Alditolen, ausgenommen Kalziumkarbonat, und Mischungen davon besteht.

2. Pulver gemäß Anspruch 1, **dadurch gekennzeichnet, dass** die durchschnittliche Partikelgröße der Granulate 25 µm bis 150 µm beträgt.

3. Pulver gemäß Anspruch 1 oder 2, **dadurch gekennzeichnet, dass** das Bindemittel eine Ballaststoff, ein Polysaccharid, ein synthetisches Polymer, ein Salz, ein Zucker, eine Aminosäure, dasselbe Material wie das Material der Primärpartikel oder Mischungen davon ist.

4. Pulver gemäß einem der Ansprüche 1 bis 3, **dadurch gekennzeichnet, dass** das Bindemittel aus einem Material hergestellt ist, das aus der Gruppe ausgewählt ist, die aus Cellulose, Hemicellulose, Methylcellulose, Cellulosederivaten, Maltodextrinen, Maisdextrinen, Gummi arabicum, Xanthan-Gummi, Guarkernmehl und Mischungen davon besteht.

5. Pulver gemäß einem der Ansprüche 1 bis 4, **dadurch gekennzeichnet, dass** die Granulate 80 bis 99,5 Gew.-% der Partikel und 0,5 bis 20 Gew.-% des Bindemittels umfassen.

6. Pulver gemäß einem der Ansprüche 1 bis 5, **dadurch gekennzeichnet, dass** die durchschnittliche Partikelgröße der Primärpartikel 5 µm bis 40 µm beträgt.

7. Pulver gemäß einem der Ansprüche 1 bis 6, **dadurch gekennzeichnet, dass** die Granulate beim Pulversprühen in Fragmente zerbrechen.

8. Pulver gemäß Anspruch 7, **dadurch gekennzeichnet, dass** das Pulver nach dem Zerbrechen eine durchschnittliche Partikelgröße von 10 µm bis 75 µm aufweist.

9. Pulver gemäß einem der Ansprüche 1 bis 8, **dadurch gekennzeichnet, dass** die Zerkleinerung in einem Pulverstrahlgerät, festgelegt durch (1-(d₅₀ nach Düse/d₅₀ bei 0,5 bar))x100 %, mindestens 20 % beträgt.

10. Pulver gemäß einem der Ansprüche 1 bis 9, **dadurch gekennzeichnet, dass** die Primärpartikel aus einem Material hergestellt sind, das aus der Gruppe ausgewählt ist, die aus Natriumhydrogencarbonat, Mannitol, Erythrit, Glycin, Trehalose, Tagatose und Mischungen davon besteht.

11. Pulver zur Verwendung zur Reinigung einer Zahnoberfläche durch Pulversprühen mit einem Pulverstrahlgerät, wobei das Pulver Granulate mit einer mittleren Partikelgröße von 20 µm bis 220 µm aufweist, **dadurch gekennzeichnet, dass** die Granulate Primärpartikel und ein Bindemittel umfassen, wobei die Primärpartikel eine mittlere Partikelgröße aufweisen, die kleiner als die mittlere Partikelgröße der Granulate ist.

12. Verfahren zur Herstellung eines Pulvers gemäß einem der Ansprüche 1 bis 10, umfassend die Schritte, dass ein Bindemittel mit Wasser gemischt wird, die resultierende Mischung auf die Primärpartikel aufgesprüht wird und die Partikel getrocknet werden, um die Granulate zu bilden.

## Revendications

1. Poudre destinée à être utilisée dans un dispositif à jet de poudre pour nettoyer la surface des dents par pulvérisation de poudre, la poudre comprenant des grains ayant une taille moyenne de 20 µm à 220 µm,
**caractérisée en ce que** les grains comprennent des particules primaires et un liant, les particules primaires ayant une taille moyenne inférieure à la taille moyenne des grains,
**caractérisée en ce que** les particules primaires sont réalisées en un matériau choisi dans le groupe constitué par les sels solubles dans l'eau, les sucres, les acides aminés, les alditols, à l'exception du carbonate de calcium, et leurs mélanges.

2. Poudre selon la revendication 1,
**caractérisée en ce que** la taille moyenne des grains est de 25 µm à 150 µm.

3. Poudre selon la revendication 1 ou 2,
**caractérisée en ce que** le liant est une fibre alimentaire, un polysaccharide, un polymère synthétique, un sel, un sucre, un acide aminé, le même matériau que celui des particules primaires, ou leurs mélanges.

4. Poudre selon l'une des revendications 1 à 3,
**caractérisée en ce que** le liant est réalisé en un matériau choisi dans le groupe constitué par la cellulose, l'hémicellulose, la méthylcellulose, les dérivés de la cellulose, les maltodextrines, les dextrines de maïs, la gomme arabique, la gomme xanthane, la gomme guar et leurs mélanges.

5. Poudre selon l'une des revendications 1 à 4,
**caractérisée en ce que** les grains comprennent 80 à 99,5 % en poids de particules et 0,5 à 20 % en poids de liant.

6. Poudre selon l'une des revendications 1 à 5,
**caractérisée en ce que** la taille moyenne des particules primaires est de 5 µm à 40 µm.

7. Poudre selon l'une des revendications 1 à 6,
**caractérisée en ce que** les grains se brisent en fragments lors de la pulvérisation de la poudre.

8. Poudre selon la revendication 7,
**caractérisée en ce que** la poudre, une fois brisée, a une taille moyenne de 10 µm à 75 µm.

9. Poudre selon l'une des revendications 1 à 8,
**caractérisée en ce que** la réduction de la taille dans un dispositif à jet de poudre, définie par (1-(d₅₀ après la buse/d₅₀ à 0,5 bar)) x 100 %, est d'au moins 20 %.

10. Poudre selon l'une des revendications 1 à 9,
**caractérisée en ce que** les particules primaires sont réalisées en un matériau choisi dans le groupe constitué par l'hydrogénocarbonate de sodium, le mannitol, l'érythritol, la glycine, le tréhalose, le tagatose et leurs mélanges.

11. Poudre à utiliser pour nettoyer la surface des dents par pulvérisation de poudre à l'aide d'un dispositif à jet de poudre, la poudre comprenant des grains ayant une taille moyenne de 20 µm à 220 µm,
**caractérisée en ce que** les grains comprennent des particules primaires et un liant, les particules primaires ayant une taille moyenne inférieure à la taille moyenne des grains.

12. Procédé de préparation d'une poudre selon l'une des revendications 1 à 10, comprenant les étapes consistant à mélanger un liant à de l'eau, à pulvériser le mélange résultant sur les particules primaires et à faire sécher les particules pour former les grains.
